(19) Europäisches Patentamt

**European Patent Office**

Office européen des brevets

(11) Veröffentlichungsnummer: **0 285 856 B1**

(12) **EUROPÄISCHE PATENTSCHRIFT**

(45) Veröffentlichungstag der Patentschrift: **26.05.93**

(51) Int. Cl.⁵: **A61K 31/48**

(21) Anmeldenummer: **88103929.1**

(22) Anmeldetag: **11.03.88**

(54) Verwendung von Dihydroergotamin und seinen Salzen zur lokalen Behandlung trophischer Störungen.

(30) Priorität: **27.03.87 DE 3710216**

(43) Veröffentlichungstag der Anmeldung:
**12.10.88 Patentblatt 88/41**

(45) Bekanntmachung des Hinweises auf die
Patenterteilung:
**26.05.93 Patentblatt 93/21**

(84) Benannte Vertragsstaaten:
**AT BE CH DE ES FR GB GR IT LI LU NL SE**

(56) Entgegenhaltungen:
**EP-A- 0 036 350     EP-A- 0 041 030**
**EP-A- 0 101 879     EP-A- 0 202 365**
**GB-A- 1 175 430     US-A- 4 451 458**

**MED.WELT, Band 26, Nr. 52, 1975, Seiten
2338-2340**

**J.SOC. COSMETICCHEMISTS, Band 21, 1971,
Seiten 299-311**

(73) Patentinhaber: **Dr. Rentschler Arzneimittel
GmbH & Co.
Postfach 320 Mittelstrasse 18
W-7958 Laupheim(DE)**

(72) Erfinder: **Seibel, Hubert, Dr.
Dahmengraben 9-13
W-5100 Aachen(DE)**

(74) Vertreter: **Schwabe - Sandmair - Marx
Stuntzstrasse 16
W-8000 München 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.6/3.3.1)

## Beschreibung

Die Erfindung betrifft die Verwendung von Dihydroergotamin und seinen Salzen zur Herstellung einer pharmazeutischen Zubereitung zur lokalen Behandlung trophischer Störungen, die sich klinisch in Form von Stauungsdermatosen, Ulcus cruris venosum und Dekubitalulcera manifestieren können.

Unter Dihydroergotamin im Sinne dieser Erfindung wird das Dihydroergotamin selbst aber auch seine pharmakologisch aktiven Stoffwechselprodukte (Metaboliten) verstanden, die aufgrund physiologischer Wechselwirkung durch Gewebe bzw. Gewebeenzyme entstehen oder isoliert werden oder synthetisiert werden, insbesondere die Hauptmetaboliten 8'−Hydroxydihydroergotamin und 8',10' − Dihydroxydihydroergotamin.

Trophische Störungen sind ernährungsbedingte und damit wachstumsbedingte Ernährungsstörungen des Gewebes oder von Organen, wobei Stauungsdermatosen und Ulcus cruris venosum schwerste Folgen einer chronisch venösen Insuffizienz, hervorgerufen durch eine konstitutionelle, anlagenbedingte Venen − wandschwäche oder Folge thrombotischer Erkrankungen sind.

Eine andere Form trophisch bedingter Hautstörungen sind Dekubitalulcera, hervorgerufen durch regio − nale Durchblutungsstörungen, inmobilisierter bettlägiger Patienten.

Aus epidemiologischen Untersuchungen geht hervor, daß etwa 2 % der Bevölkerung aus Industrielän − dern Ulcera im Bereich der Beine aufweisen, wovon ca. 90 % venös bedingt sind (Schmidli et al., Pharmakritik, 8, 6, 1986, S. 21 − 24). Daraus wird deutlich, daß neben der eingeschränkten Lebensqualität für den Betroffenen auch erhebliche volkswirtschaftliche Verluste durch Krankenhausaufenthalte und Ar − beitsunfähigkeit entstehen. Zur Behandlung von Ulcus cruris venosum, Stauungsdermatosen und Dekubital ulcera ist bislang keine eindeutige medikamentöse Standardtherapie bekannt geworden. Vielmehr werden diese Krankheiten polypragmatisch durch symptomatische Behandlung therapiert, mit letztlich ungewissen Erfolgen. Nach der Schulmedizin umfaßt die Therapie die Reinigung des Ulcus auf mechanischem, osmotischem oder enzymatischem Wege und eine gleichzeitige antimikrobielle und antiphlogistische Behandlung sowie die Verwendung granulationsfördernder und epithelisierender Mittel (M. Mörl, Fortschr. Med. 104, 1986, Nr. 21), zumeist unter Anlegen von Druckverbänden, um den venösen Abfluß auf physikalischem Weg zu erreichen und Ruhigstellung. Die Zweifelhaftigkeit dieser Behandlungsmethoden beschreiben Schmidli und Holzer in "Lokale Behandlung von Hautulcera" in Pharmakritik, 8, 6, 1986.

Die Verwendung von Dihydroergotamin und seinen Salzen in Arzneiformen zur peroralen und parente − ralen Anwendung zur Gefäßtonisierung, um die hypotone orthostatische Dysregulation (niedriger Blut − druck), den Migräneanfall und die chronisch venöse Insuffizient zu behandeln sowie Migräneprophylaxe zu betreiben, ist bekannt.

Eine lokale Anwendung von Dihydroergotamin zur Behandlung trophischer Störungen wie zum Beispiel der Stauungsdermatose, des Ulcus cruris venosum und des Dekubitalulcus war bislang nicht bekannt. Dafür vorgesehene oral einzunehmende Lösungen sind z. B. aus der EP − A − 101 879 bekannt.

Es wurde überraschend gefunden, daß die vorgenannten Erkrankungen mit Erfolg behandelt werden können, wenn, entgegen der klassichen Verwendung von Dihydroergotamin und seinen Salzen, dieses lokal angewendet wird. Es war darüber hinaus überraschend, daß die nassen und nässenden offenen Wunden bei Ulcerationen bereits nach kurzer Zeit unter Einwirkung auch von flüssigen Darreichungsformen ab − trocknen. Dabei kann auf die Anwendung von Antibiotika und Reinigungsmitteln zur Bekämpfung bakterieller Infektionen, die üblicherweise auftreten und die Behandlung erschweren, völlig verzichtet werden. Es war weiter überraschend, daß bereits nach kurzer Zeit, bezogen auf die übliche Therapiedauer, Gefäßeinspros − sungen aus der Tiefe des Ulcus sowie deutlich sichtbare Granulationsinseln zu beobachten sind.

Ferner wurde überraschend festgestellt, daß die Heilung sowohl aus der Tiefe des Ulcus wie vom Rand her durch Gewebszuwachs erfolgt, so daß in der Regel nachfolgend keine kosmetischen Korrekturen erforderlich sind. Dabei bleiben objektiv zu erwartende Nebenwirkungen, wie z. B. Blutdruckanstieg, aus.

Stauungsdermatosen und postthrombotische Dermatitiden, die durch auffallende und oft großflächige mittel − bis dunkelbraune Verfleckungen gekennzeichnet sind, zeigen überraschenderweise nach Anwen − dung von Dihydroergotamin bereits nach kurzer Zeit eine deutliche Aufhellung der befallenen Hautbezirke. Nach mehrwöchiger Behandlungszeit hat die Haut sich hinsichtlich Farbe und Elastizität wieder dem normalen Umfeld angepaßt.

Die Verwendung von Dihydroergotamin und seinen Salzen, wie zum Beispiel Methansulfonat, Äthan − sulfonat, Tartrat, Maleat, Succinat und andere für die lokale Anwendung geeigneter Salze zur Herstellung einer pharmazeutischen Zubereitung zur lokalen Behandlung vorgenannter trophischer Störungen kann in Form flüssiger, halbfester oder fester Arzneimitteln erfolgen, sowie eben solcher Arzneimittel, welche die aktiven Metaboliten des Dihydroergotamins, z. B. 8'−Hydroxydihydroergotamin und 8',10'−Dihydroxydih − ydroergotamins allein oder zusammen mit Dihydroergotamin enthalten.

Flüssige Arzneimittel in diesem Sinne sind Lösungen in Form von Tropfen, Tinkturen und Sprays, Suspensionen, Emulsionen, Dispersionen. Als halbfeste Arzneiformen kommen zum Beispiel Gele, Salben, Cremes und Schäume in Frage, während unter festen Arzneiformen beispielsweise Pulver, Puder, Granulate, Pellets und Mikrokapseln verstanden werden.

Die Arzneiformen enthalten möglichst reizfreie Verdünnungsmittel, von denen neben Wasser, einwertige Alkohole, mehrwertige Alkohole, Polyglykole, aber auch Glycerinformal, Dimethylisosorbid, natürliche und synthetische Öle und Ester verwendet werden können. Ethanol, Glycerin, Propandiol, Polyethylenglykole, Miglyol [R] als einige typische Vertreter seien beispielhaft genannt. Für die Herstellung von halbfesten Darreichungsformen eignen sich neben vorgenannten Lösungsmitteln zusätzlich Grundmassen wie zum Beispiel Bentonit, Veegum, Guarmehl und Cellulosederivate wie Methylcellulose, Carboxymethylcellulose aber auch Polymere aus Vinylalkohol und Vinylpyrrolidon, Alginate, Pektine, Polyacrylate, feste und flüssige Polyethylenglykole, Paraffine, Fettalkohole, Vaseline und Wachse, Fettsäuren und Fettsäureester. Feste Arzneiformen enthalten entweder den Wirkstoff unverdünnt oder mit Verdünnungsmitteln wie beispielsweise kolloidale Kieselsäure, Talkum, Milchzucker, Stärkepulvern, Zuckern, Cellulosederivaten und Gelatine, Metalloxide und Metallsalze. Die Arzneiformen können darüber hinaus noch andere Bestandteile wie Konservierungsmittel, Stabilisatoren, Tenside, Emulgatoren und Penetrationsförderer, Spreitungsmittel, Puffersubstanzen und Treibmittel enthalten. Die Verwendung der beispielhaft genannten Verdünnungsmittel und Hilfsstoffe ist nicht arzneiformspezifisch. Sie können wahlweise für verschiedene Formen Einsatz finden. Besonders bevorzugt werden Zusammensetzungen, die aufgrund ihrer Stoffeigenschaften Autosterilität garantieren, wobei dann auf zusätzliche Konservierung verzichtet werden kann. Geeignet sind dabei Zusammensetzungen, die mehr als 10 Gew.−% Propylenglykol und/oder Glycerin oder Mischungen davon enthalten und sich zusätzlich durch eine relativ gute Verträglichkeit auszeichnen.

Die Verfahren zur Herstellung der flüssigen und halbfesten Formen sind vorzugsweise so zu wählen, daß der Wirkstoff überwiegend oder vollständig gelöst oder gleichmäßig verteilt vorliegt, um seine Wirkung unmittelbar zu entwickeln.

Die Verfahren zur Herstellung der Arzneiformen sind an sich bekannt. Zusätzliche Forderung an die Verfahren ist jedoch, daß insbesondere die flüssigen und halbfesten Darreichungsformen nach der Herstellung und Abfüllung bzw. Verpackung keimfrei sind, um eine Sekundärinfizierung der zu behandelnden erkrankten Flächen zu verhindern. Mindestanforderung ist, daß die Arzneiformen weniger als 1000, vorzugsweise weniger als 100 vermehrungsfähiger, nichtpathogener Mikroorganismen enthalten.

Der pH−Wert flüssiger und halbfester Arzneiformen ist so einzustellen und gegebenenfalls zu korrigieren, daß er den physiologischen Verhältnissen nahekommt. Dabei ist ein pH−Bereich von 3,5 bis 8,5, vorzugsweise 5 bis 7,8, anzustreben.

Darüber hinaus kann die Verwendung auch mittels präparierter Gazestreifen, Kompressen und Pflaster erfolgen, die mit den vorgenannten Arzneiformen oder Kombinationen davon getränkt bzw. belegt wurden.

Die Wirkstoffkonzentration in den Arzneimittelzusammensetzungen ist nicht kritisch und beträgt zwischen 0,001 und 100 Gew.−%, in den flüssigen und halbfesten Arzneiformen vorzugsweise etwa 0,01 bis 5 Gewichtsprozent, in den festen Formen vorzugsweise 0,01 bis 100 Gewichtsprozent, d. h. der reine Wirkstoff kann unter Verwendung entsprechender Dosierhilfsmittel zur genauen Dosierung unverdünnt angewendet werden. Die Differenz zu 100 Gewichtsprozent ergibt sich aus dem jeweiligen Anteil Verdünnungsmittel bzw. den verwendeten Hilfsstoffen.

Die Abfüllung der Arzneiformen erfolgt in an sich bekannten Behältnissen wie Flaschen, Tuben, Puder- und Streudosen, Siegelrandbeuteln, die gegebenenfalls mit Dosierungshilfen wie Tropfern, Dosierventilen und Dosierkammern ausgestattet sind.

**Beispiel 1**

Eine 56−jährige Patientin, die seit 42 Jahren am linken Unterschenkel in Höhe des Knöchels an einem Ulcus cruris venosum litt, galt nach Behandlung mittels gängiger Therapieprinzipien einschließlich erfolglos verlaufender Hauttransplantation als klinisch austherapiert.

Das Ulcus war zu Beginn der lokalen Behandlung unter Verwendung von Dihydroergotamin etwa 10 x 6 cm groß bei einer Tiefe von 0,5 bis 1 cm. Die Wunde juckte, näßte, verbreitete üblen Geruch und war von schmierigen, gelblichen Belägen bedeckt. Die Wunde war mit einer etwa dreifach so großen Stauungsdermatose umgeben. Zu Therapiebeginn war in der Tiefe der Wunde der Unterschenkelknochen erkennbar. Die Behandlung erfolgte derart, daß eine Lösung nach Beispiel 4 zunächst direkt auf die Wunde aufgeträufelt wurde. Später wurden mit der Lösung getränkte Gazestreifen direkt auf die Wunde gelegt. Dabei wurde die Lösung so dosiert, daß etwa 0,1 mg Dihydroergotamin pro cm$^2$ Wundfläche zur Verfügung standen.

Bereits nach mehrtägiger lokaler Behandlung der Wunde verschwand das Jucken, Nässen und der üble Geruch, gefolgt von der Bildung randständigen Granulationsgewebes, Granulationsinseln und Gefäßeinsprossungen nach mehreren Wochen.

Nach viermonatiger Therapie war das Ulcus auf Streichholzschachtelgröße verkleinert und ein deutlich deckender Gewebszuwachs aus der Tiefe erkennbar. Nach weiteren drei Monaten Behandlungszeit war die Wunde narbig, völlig ausgeheilt. Die Zeichen der Stauungsdermatose waren fast völlig verschwunden.

Dieser Behandlungserfolg ist umso erstaunlicher, da bei der Patientin die denkbar schlechtesten Voraussetzungen für eine Heilung vorlagen (Übergewicht, Stehberuf, Raucherin, jahrzehntelange Anamnese).

**Beispiel 2**

Bei einer 75–jährigen Patientin mit schwerer Cerebralsklerose und Herzfehler wurden mehrere Ulcerationen behandelt. Ein Ulcus im Bereich des Kreuzbeins hatte eine größe von 7 x 5 cm. Kleinere Ulcerationen wurden im Bereich beider Schulterblätter und beider Fersen festgestellt. Die Therapie erfolgte mit vorpräparierten Kompressen der erfindungsgemäßen Darreichungsformen. Die Heilung der Schulterulcera war nach drei Wochen, die des Kreuzbeinulcus nach fünf Wochen und die Fersenulcera nach drei bzw. fünfeinhalb Wochen abgeschlossen. Es kam jeweils zum völligen Wunderverschluß.

**Beispiel 3**

Ein 50–jähriger männlicher Patient litt seit 12 Jahren an intensiven mittel– bis dunkelbraunen Verfärbungen, Kennzeichen einer Stauungsdermatitis oder postthrombotischen Dermatose besonders am linken Unterschenkel im tibialen Bereich.

Dem Patienten wurde aufgetragen, nach sorgfältigen Waschungen, die verfleckten Bezirkte mit einer dihydroergotaminhaltigen Darreichungsform der erfindungsgemäßen Art einzureiben (Gel). Bereits nach 14 Tagen konnte eine deutliche Aufhellung der Hautbezirke beobachtet werden. Nach zehn Wochen Therapiedauer war eine völlige Normalisierung der Haut in Farbe und Elastizität erreicht.

Die nachfolgenden Beispiele geben Zusammensetzungen von Arzneiformen und Verfahren zu ihrer Herstellung an, die zur lokalen Behandlung trophischer Störungen mit Dihydroergotamin geeignet sind.

**Beispiel 4** (Lösung)

| | |
|---|---|
| Dihydroergotaminmethansulfonat | 2,00 g |
| Propylenglykol | 556,60 g |
| Glycerin | 52,50 g |
| Wasser | 438,90 g |
| | 1 050,00 g |

Dihydroergotaminmethansulfonat wird unter Rühren in der Mischung aus Propylenglykol und Glycerin gelöst. Nach Zugabe von Wasser wird die Lösung sterilfiltriert und in Flaschen abgefüllt.
Die Lösung enthält 2,0 mg Wirkstoff pro ml Lösung.
Die Flaschen werden mit einem Tropfer versehen, der es erlaubt je Tropfen 0,1 mg Wirkstoff zu dosieren.

4

**Beispiel 5** (Gel)

| | |
|---|---|
| Dihydroergotaminmethansulfonat | 2,00 g |
| Propylenglykol | 700,00 g |
| Bentonit (Bentone LT) | 29,00 g |
| Glycerin | 45,00 g |
| Wasser | 224,00 g |
| | 1 000,00 g |

Bentone LT wird hitzesterilisiert und dann in einem Teil Propylenglykol suspendiert. In die Suspension wird zunächst Glycerin und Wasser eingearbeitet und anschließend eine Lösung von Dihydroergotamin‑methansulfonat in dem restlichen Propylenglykol.

Die Herstellung erfolgt bei Raumtemperatur unter Vakuum bei keimfreien Bedingungen. Es wird ein gut streichbares Gel mit einer Viskosität von ca. 32.000 Centipoise und einem pH‑Wert von 7,6 erhalten, welches in innenschutzlackierte Tuben abgefüllt wird.

**Beispiel 6** (Gel)

| | |
|---|---|
| Dihydroergotaminmethansulfonat | 4,00 g |
| Propylenglykol | 500,00 g |
| Natriumcarboxymethylcellulose | 17,00 g |
| Glycerin | 45,00 g |
| Wasser | 433,83 g |
| Propylgallat | 0,05 g |
| Thiodipropionsäure | 0,10 g |
| Titriplex III | 0,02 g |
| | 1 000,00 g |

Natriumcarboxymethylcellulose wird in einem Teil Propylenglykol vorsuspendiert. In die Suspension wird eine Lösung aus Glycerin, Wasser, Propylgallat, Thiodipropionsäure und Titriplex III eingerührt. Zuletzt wird das in der restlichen Menge Propylenglykol gelöste Dihydroergotaminmethansulfonat zu der Mischung gegeben.

Die Herstellung erfolgt bei Raumtemperatur unter Vakuum bei keimfreien Bedingungen.

5

Es wird ein gut streichbares Gel mit einer Viskosität von ca. 32.000 Centipoise und einem pH−Wert von 6,3 erhalten. Auf eine sterile, mehrlagige Gaze mit einer Fläche von ca. 100 cm$^2$ werden 5 g Gel aufgetragen, gleichmäßig verteilt, mit einer Deckfolie abgedeckt und in einem Siegelrundaluminiumbeutel eingesiegelt.

Die anwendungsfertige Gaze enthält 0,2 mg Wirkstoff pro Quadratzentimeter.

**Beispiel 7** (Pulver)

| | |
|---|---|
| Dihydroergotaminmethansulfonat | 10,0 g |
| Milchzucker | 188,0 g |
| Kolloidale Kieselsäure | 2,0 g |
| | 200,0 g |

Dihydroergotaminmethansulfonat und Milchzucker werden unter Verwendung eines Wasser/Ethanol−Gemisches feucht granuliert. Anschließend wird das Lösungsmittelgemisch abgedampft und das grobe Granulat mittels einer Granuliermaschine auf eine Korngröße von kleiner 100 $\mu$ zerkleinert. Nachdem die Kieselsäure zugemischt ist, wird das Pulver in eine Streudose gefüllt.

Das Pulver kann bedarfsgerecht aufgestreut werden, wobei die Wirkstoffkonzentration von 0,05 g pro g Pulver einen sehr dünnen Auftrag erlaubt.

**Beispiel 8**

| | |
|---|---|
| Dihydroergotaminmethansulfonat | 10,0 g |
| Propylenglykol | 3.515,0 g |
| Hydroxyethylcellulose | |
| (Natrosol $^{(R)}$ 250 HX) | 60,0 g |
| Glycerin | 225,0 g |
| Wasser | 1.190,0 g |
| | 5.000,0 g |

2000 g Propylenglykol werden auf 60 °C erwärmt. Darin wird die Hydroxyethylcellulose unter Rühren suspendiert. In die Suspension wird eine Mischung aus 225 g Glycerin und der angegebenen Menge Wasser gegeben und 1 Stunde gerührt. Anschließend wird auf Raumtemperatur abgekühlt und eine Lösung aus Dihydroergotamin in 1515 g Propylenglykol eingerührt. Unter Vakuum wird 1 Stunde nachgerührt.

Die Herstellung erfolgt unter keimarmen Bedingungen. Es wird ein gut streichbares, klares Gel mit einer Viskosität von 10.500 Centipoise und einem pH−Wert von 5,5 erhalten.

Das Gel wird in Aluminiumtuben, die mit einem Innenschutzlack versehen sind, zu 30 g abgefüllt.

### Beispiel 9

Bei einer 39 – jährigen Patientin wurde eine Varikosis beider Unterschenkel sowie ein kreisrundes Ulcus am linken Unterschenkel, ca. 1 cm Durchmesser und 4 mm tief, diagnostiziert. Das Ulcus war von einer 5 mm breiten Hautrötung umgeben; schmieriger Wundbelag.

Das Ulcus wurde mit einem Dihydroergotamin – Gel, gemäß Beispiel 8 behandelt. Das Gel wurde auf den Ulcusboden und der Umgebung dünn aufgetragen, mit Mull abgedeckt. Der Verband wurde täglich erneuert. Nach 2 Tagen war Wundreinigung erfolgt, nach 7 Tagen die Hautrötung abgeklungen. Nach 2 Monaten war das Ulcus geschlossen und dünn überhäutet.

### Beispiel 10

Bei einer 59 – jährigen Patientin wurde am linken Oberschenkel ein rechteckiges Ulcus cruris diagon – stiziert. Die Wunde nahm eine Fläche von ca. 4 x 2,5 cm ein und war 3 mm tief. Sie war von einem breiten, stark roten Wundsaum umgeben. Wundnischen und Ulcusboden waren mit einem schmierigen, stark riechenden Belag bedeckt.

Dihydroergotamin – Gel, gemäß Beispiel 8, wurde auf Ulcusboden und Wundrändern dünn, in den Wundnischen dicker aufgetragen. Die Wunde wurde mit Mull abgedeckt. Es erfolgte täglicher Verbands – wechsel. Die Wunde war nach 3 Tagen gereinigt. Nach 7 Tagen war die Hautrötung abgeklungen. Nach 5 Tagen war eine deutliche Granulation in den Wundnischen (leichte, hellrote Blutung aus Ulcusboden) sichtbar. Die Wundnischen waren nach 14 Tagen geschlossen. Das Ulcus war nach 10 Wochen abgeheilt und dünn überhäutet.

### Beispiel 11

Bei einer 62 – jährigen Patientin wurde eine Varikosis beider Beine, venöse Ödeme beider Füße, Lymphödeme beider Arme und Hände diagnostiziert. Am linken Oberschenkel, dicht oberhalb des Sprunggelenkes, befand sich ein Ulcus cruris von 1,5 cm Durchmesser und 4 mm Tiefe. Die umgebende Haut war reizlos, die Wunde war relativ sauber.

Dihydroertoamin – Gel gemäß Beispiel 8 wurde dünn auf den Wundboden aufgetragen, die Wunde mit Mull abgedeckt. Es erfolgte täglicher Verbandswechsel. Nach 12 Wochen war die Wunde mit einer zarten Haut verschlossen.

### Beispiel 12

Eine 89 – jährige Patientin litt über dem Kreuzbein an einem Dekubitalulcus mit einer Fläche von 3 x 2 cm. Der Ulcusboden war schmierig.

Dihydroergotamin – Gel gemäß Beispiel 8 wurde dünn aufgetragen, das Ulcus mit Mull abgedeckt. Es erfolgte täglicher Verbandswechsel. Nach 2 Tagen war die Wunde gesäubert. Nach 4 Wochen wurde Wundverschluß mit dünner Hautdecke festgestellt.

### Patentansprüche

1. Verwendung von Dihydroergotamin und/oder seinen aktiven Metaboliten, vorzugsweise 8' – Hydrox – ydihydroergotamin und 8', 10' – Dihydroxydihydroergotamin zur Herstellung einer pharmazeutischen Zubereitung zur lokalen Behandlung von Stauungsdermatosen, Ulcus cruris venosum und Dekubitalul – cera.

2. Verwendung nach Anspruch 1, dadurch gekennzeichnet, daß die Zubereitung 0,001 bis 100 Gew. – % Dihydroergotamin oder eines seiner Salze und/oder einen aktiven Metaboliten enthält.

3. Verwendung nach Anspruch 2, dadurch gekennzeichnet, daß die Zubereitung flüssig ist und zwischen 0,01 und 5 Gew. – % Dihydroergotamin und/oder eines seiner Salze und zwischen 99,99 und 95 Gew. – % Verdünnungsmittel enthält.

4. Verwendung nach Anspruch 2, dadurch gekennzeichnet, daß die Zubereitung halbfest ist und zwischen 0,01 und 5 Gew. – % Dihydroergotamin und/oder eines seiner Salze und zwischen 99,99 und 95 Gew. – % Verdünnungsmittel enthält.

**5.** Verwendung nach Anspruch 2, dadurch gekennzeichnet, daß die Zubereitung fest ist und zwischen 0,01 und 100 Gew.−% Dihydroergotamin und/oder eines seiner Salze und zwischen 0 bis 99,99 % Verdünnungsmittel enthält.

**6.** Verwendung nach Anspruch 3 oder 4, dadurch gekennzeichnet, daß die Zubereitung mehr als 10 Gew.−% Propylenglykol und/oder Glycerin oder deren Mischungen enthält.

**7.** Verwendung nach einem der Ansprüche 1 bis 5, dadurch gekennzeichnet, daß in der Zubereitung ein pH−Wert von 3,5 bis 8,5 eingestellt wird.

**8.** Verwendung nach Anspruch 3 oder 4, dadurch gekennzeichnet, daß der Wirkstoff Dihydroergotamin bzw. seine Salze in der Zubereitung überwiegend oder vollständig gelöst ist (sind).

**9.** Verwendung nach einem der Ansprüche 1 bis 8, dadurch gekennzeichnet, daß die Zubereitung auf entsprechenden Trägermaterialien aufgebracht ist.

## Claims

**1.** Use of dihydroergotamine and/or its active metabolites, preferably 8'−hydroxydihydroergotamine and 8',10,−dihydroxydihydroergotamine for the making of a pharmaceutical preparation for the local treatment of venostatic dermatoses, varicose ulcers and decubital ulcers.

**2.** Use according to claim 1, characterized in that the preparation contains 0.001 to 100% by weight dihydroergotamine or one of its salts and/or an active metabolite.

**3.** Use according to claim 2, characterized in that the preparation is liquid and contains between 0.01 and 5% by weight dihydroergotamine and/or one of its salts and between 99.99 and 95% by weight diluting agent.

**4.** Use according to claim 2, characterized in that the preparation is semi−solid and contains between 0.01 and 5% by weight dihydroergotamine and/or one of its salts and between 99.99 and 95% by weight diluting agent.

**5.** Use according to claim 2, characterized in that the preparation is solid and contains between 0.01 and 100% by weight dihydroergotamine and/or one of its salts and between 0 to 99.99% diluting agent.

**6.** Use according to claims 3 or 4, characterized in that the preparation contains more than 10% by weight propylene glycol and/or glycerin or mixtures thereof.

**7.** Use according to one of claims 1 to 5, characterized in that a pH value of 3.5 to 8.5 is adjusted in the preparation.

**8.** Use according to claim 3 or 4, characterized in that the active ingredient dihydroergotamine or its salts is or are mainly or completely dissolved in the preparation.

**9.** Use according to one of claims 1 to 8, characterized in that the preparation is applied to suitable carrier materials.

## Revendications

**1.** Utilisation de dihydroergotamine et/ou de ses métabolites actifs, de préférence de la 8'−hydroxydihydroergotamine et de la 8',10'−dihydroxydihydroergotamine pour la mise au point d'une préparation pharmaceutique destinée au traitement local de dermatoses par stase veineuse, de l'Ulcus cruris venosum et de l'ulcère décubital.

**2.** Utilisation selon la revendication 1, caractérisée en ce que la préparation contient de 0,001 à 100 % en poids de dihydroergotamine ou d'un de ses sels et/ou un métabolite actif.

3. Utilisation selon la revendication 2, caractérisee en ce que la préparation est liquide et contient entre 0,01 et 5 % en poids de dihydroergotamine et/ou d'un de ses sels et entre 99,99 et 95 % en poids d'un diluant.

4. Utilisation selon la revendication 2, caractérisée en ce que la préparation est semi‒solide et contient entre 0,01 et 5 % en poids de dihydroergotamine et/ou d'un de ses sels et entre 99,99 et 95 % en poids d'un diluant.

5. Utilisation selon la revendication 2, caractérisée en ce que la préparation est solide et contient entre 0,01 et 100 % en poids de dihydroergotamine et/ou d'un de ses sels et entre 0 et 99,99 % en poids d'un diluant.

6. Utilisation selon la revendication 3 ou 4, caractérisée en ce que la préparation contient plus de 10 % en poids de propylèneglycol et/ou de glycérine ou de mélanges de ces derniers.

7. Utilisation selon l'une quelconque des revendications 1 à 5, caractérisée en ce que, dans la prépara‒tion, on règle une valeur de pH de 3,5 à 8,5.

8. Utilisation selon la revendication 3 ou 4, caractérisée en ce qu'on dissout la substance active dihydroergotamine ou ses sels principalement ou complètement dans la préparation.

9. Utilisation selon l'une quelconque des revendications 1 à 8, caractérisée en ce qu'on applique la préparation sur des matières de support correspondantes.